# EUROPÄISCHE PATENTSCHRIFT

(11) **EP 1 309 537 B1**
(45) Veröffentlichungstag und Bekanntmachung des Hinweises auf die Patenterteilung: **16.11.2005**
(21) Anmeldenummer: 01960489.1
(22) Anmeldetag: 11.07.2001
(51) Int. Cl.: C07C 51/265, C07C 51/25, C07C 57/05, C07C 63/16, C07C 63/26, B01J 8/04, B01J 8/18, B01J 8/02

(54) **VERFAHREN ZUM KATALYTISCHEN ERZEUGEN VON ORGANISCHEN STOFFEN DURCH PARTIELLE OXIDATION**
METHOD FOR CATALYTICALLY PRODUCING ORGANIC SUBSTANCES BY PARTIAL OXIDATION
PROCEDE DE PRODUCTION CATALYTIQUE DE MATIERES ORGANIQUES PAR OXYDATION PARTIELLE

(30) Priorität: 09.08.2000 DE 10038755; 01.02.2001 DE 10104406
(43) Veröffentlichungstag der Anmeldung: 14.05.2003
(73) Patentinhaber: MG Technologies AG, 60325 Frankfurt am Main (DE)
(72) Erfinder: FRANZ, Volkev, 60486 Frankfürt/Main (DE); DOMES, Helmuth, 63179 Obertshaüsen (DE)
(86) Internationale Anmeldenummer: PCT/EP2001/008002
(87) Internationale Veröffentlichungsnummer: WO 2002/012158

(56) Entgegenhaltungen:
- EP-A- 0 938 924
- GB-A- 728 821
- DATABASE WPI Section Ch, Week 197801 Derwent Publications Ltd., London, GB; Class E35, AN 1978-00874A XP002195799 & JP 52 136880 A (RICOH KK), 15. November 1977 (1977-11-15)

## Beschreibung

Die Erfindung betrifft ein Verfahren zum katalytischen Erzeugen von organischen Stoffen durch partielle Oxidation eines organischen Einsatzstoffes in Gegenwart von molekularem Sauerstoff bei Temperaturen im Bereich von 200 bis 500 °C in einem oder mehreren, in Serie geschalteten, einen Katalysator enthaltenden Reaktoren zur Erzeugung eines gasförmigen Produktgemisches.

Die Umwandlung des Einsatzstoffes in das Endprodukt erfolgt hierbei in einem oder mehreren Oxidationsschritten, wobei die einzelnen Moleküle des Einsatzstoffes bei jedem Oxidationsschritt ein oder mehrere Atome Wasserstoff und/oder Kohlenstoff abgeben und auch einzelne Sauerstoffatome binden können. Der abgegebene Kohlenstoff verbindet sich dabei mit Sauerstoff zu Kohlendioxid und/oder Kohlenmonoxid, der abgegebene Wasserstoff zu Wasser. Ein Teil des Einsatzproduktes wird hierbei üblicherweise zu Kohlendioxid und/oder Kohlenmonoxid total oxidiert, ein anderer Teil zu anderen organischen Stoffen umgewandelt. Der für die Oxidationsreaktion benötigte Sauerstoff wird aus dem Trägergas (üblicherweise in einem Wärmeaustauscher vorgewärmte Umgebungsluft) entnommen. Bedingt durch die verschiedenen Oxidationsreaktionen ist das gesamte Verfahren stark exotherm. Erfindungsgemäß wird z.B. Phthalsäureanhydrid, Maleinsäureanhydrid aus Butan oder Benzol, Acrylsäure aus Propylen, Essigsäure aus C₄-Kohlenwasserstoffen oder Anthrachinon erzeugt.

Ein typisches und weltweit in großer Menge nach diesem Prinzip erzeugtes Produkt ist Phthalsäureanhydrid (PSA) aus einem Einsatzgemisch, welches Orthoxylol oder Naphthalin und molekularen Sauerstoff in der für Umgebungsluft üblichen oder durch zusätzliche Anreicherung erhöhten Konzentration enthält. Die Erzeugung des PSA geschieht bei Temperaturen im Bereich von 200 bis 500 °C in einem Katalysator enthaltenden, von Kühlfluid durchströmten Reaktor, aus welchem ein gasförmiges, PSA- Dampf enthaltendes Produktgemisch abgezogen wird. Diese PSA-Erzeugung wird im weiteren beispielhaft für alle durch katalytische partielle Oxidation in der Gasphase erzeugbaren organischen Produkte dargestellt, wobei man auch mehrere, in Serie geschaltete Reaktoren einsetzen kann.

Ein Verfahren dieser Art mit einem Vielrohrreaktor ist aus dem US-Patent 4 592 412 bekannt. Hierbei enthält der Reaktor eine Vielzahl vertikaler Rohre, in welchem sich der körnige Katalysator befindet. Der Katalysator auf Vanadiumpentoxid- Basis kann auch aus mit katalytischer Masse beschichteten Trägerkörpern gebildet sein. Die zahlreichen, den Katalysator enthaltenden Rohre sind auf ihrer Außenseite von einem Kühlfluid umströmt, um die durch die exotherme Reaktion frei werdende Wärme abzuführen. Beim bekannten Verfahren verwendet man vorzugsweise eine Salzschmelze als Kühlfluid, welche aber aufwendige und dadurch teure Apparaturen notwendig macht.

Neben hohen Investitionskosten ist der vorbeschriebene Vielrohrreaktor auch aus verschiedenen anderen Gründen problematisch. Durch hohes Gewicht und große Abmessungen des Reaktors (150 bis 250 und 7 bis 9 m Durchmesser allein für das Rohrbündel bei üblichen Reaktorgrößen) sind kostenintensive Spezialtransporte erforderlich. Erforderliches Vormaterial in nicht handelsüblichen Abmessungen sowie arbeitsintensive Fertigung bedingen lange Lieferzeiten bei der Reaktorherstellung. Die regellose Schüttung des Katalysators in den einzelnen Rohren verursacht einen großen gasseitigen Druckverlust verbunden mit einem hohen Energieaufwand für die Förderung des Einsatzgases. Außerdem sind mit dieser Bauart eine Reihe weiterer, meist verfahrenstechnisch oder reaktionstechnisch bedingter Probleme verbunden, auf die im folgenden näher eingegangen wird.

Moderne Hochleistungskatalysatoren erfordern sehr gleichmäßige Temperaturen zur Entfaltung hoher Selektivität und Erzielung einer langen Lebensdauer. Die zulässige Temperaturabweichung des Kühlfluids innerhalb des die Rohre umgebenden Raumes ist daher eng begrenzt. Dies erfordert beim bekannten Verfahren große Umwälzmengen des Kühlfluids verbunden mit hohem Energie- und Investitionskostenaufwand für dessen Umwälzpumpen.

Typischerweise erfolgt bei allen bekannten Oxidationsverfahren, die mit einem Reaktor mit ortsfest eingebautem Katalysator (Festbett) arbeiten, die Umsetzung des Einsatzmaterials in das Endprodukt und die damit verbundene Wärmeentwicklung über die gesamte Katalysatorfüllung sehr ungleichmäßig, denn man stellt fest, dass beispielsweise beim PSA-Prozess mehr als 90 % der Reaktionswärme auf die eintrittsseitige Hälfte der Katalysatorfüllung (Hauptreaktionszone) entfallen. Beim Reaktor nach dem bekannten Verfahren bilden sich, bedingt durch schlechten Wärmetransport zwischen Katalysator und Rohrwand, im Katalysator der Hauptreaktionszone Reaktionstemperaturen weit oberhalb der Temperatur der Rohrwand aus, während in der austrittsseitigen Hälfte der Katalysatorfüllung (Nachreaktionszone) die Reaktionstemperatur sehr nahe bei der des Kühlfluids liegt. Meist bildet sich in der eintrittsseitigen Katalysatorhälfte ein sogenannter hot spot aus, dessen Temperatur nahe an die Selbstentzündungstemperatur des Produkts reichen kann. Damit ist selbst bei geringfügigen Schwankungen der Reaktionsbedingungen die Gefahr einer Zündung des Gases mit teilweiser oder totaler Schädigung des Katalysator durch Überhitzung gegeben. Bei hohen Temperaturen im Katalysator kann außerdem ein Rückkoppelungseffekt den Prozess der Überhitzung beschleunigen, denn mit einer Erhöhung der Reaktionstemperatur steigt erfahrungsgemäß auch die Aktivität des Katalysators und damit auch die Menge oxidierten Einsatzproduktes, wobei die zusätzliche Wärmeerzeugung leicht zum Erreichen der Selbstentzündungs- Temperatur des Gases führen kann. Mit der Steigerung der Aktivität sinkt außerdem die Selektivität des Katalysators, d.h. die Produktausbeute nimmt ab und es werden mehr Nebenprodukte gebildet.

Für die Wirtschaftlichkeit der PSA-Herstellung ist es vorteilhaft, ein Reaktor- Einsatzgas mit einer möglichst hohen Konzentration an Einsatzstoffen zu verwenden. Hierbei können für Anlagen gleicher Leistung kleinere und damit billigere Apparaturen eingesetzt werden. Beim bekannten Verfahren behindert die vorbeschriebene Gefahr der Überreaktion des Katalysators jedoch Steigerungen der Konzentration in dem für die Wirtschaftlichkeit wünschenswertem Maße.

Der Erfindung liegt die Aufgabe zugrunde, das eingangs genannte Verfahren weiter zu entwickeln und bei verbesserter Betriebssicherheit und kostengünstigerem Betrieb die Produktausbeute zu steigern. Hierbei soll nicht nur mit einem Reaktor sondern auch mit mehreren, in Serie geschalteten Reaktoren gearbeitet werden können, um z.B. vorhandene Anlagen erweitern zu können.

Erfindungsgemäß gelingt dies dadurch, dass mindestens ein Reaktor als Kühlreaktor mit von Kühlfluid durchströmten Kühlrohren ausgebildet ist, wobei im Kühlrohrreaktor 40 bis 100 Gew.-% der Gesamtmenge des Katalysators auf der Außenseite der Kühlrohre als Beschichtung angeordnet ist und das den Einsatzstoff und den molekularen Sauerstoff enthaltende Einsatzgemisch mit den Katalysatorschichten in Kontakt kommt und dass mehrere Kühlsysteme vorhanden sind, welche Kühlfluid zu den Kühlrohren des Kühlreaktors fördern und daraus abziehen. Ein Teil der gesamten Katalysatormenge kann auf ungekühlten Flächen im Kühlreaktor angebracht sein.

Ein weiterer wesentlicher Aspekt der Erfindung besteht darin, dass zur Abführung der Reaktionswärme im Kühlrohrreaktor eine verdampfbare Flüssigkeit anstelle der beim bekannten Röhrenreaktor üblichen Salzschmelze verwendet wird, z.B. kann man Diphyl als Kühlfluid verwenden. Nachfolgend wird teilweise die Herstellung von PSA angesprochen, doch gelten die Erläuterungen analog auch für die Herstellung anderer Stoffe.

Vorzugsweise bildet man im Kühlreaktor mindestens die Hälfte der Kühlrohre als Rippenrohre mit an der Außenseite vorspringen den Rippen aus, wobei die Rippen teilweise mit Katalysator beschichtet sind. Durch die Verwendung von Rippen rohren anstelle von glatten Rohren lässt sich die Katalytisch aktive Oberfläche erheblich vergrößern, ohne den Rohrdurchmesser oder die Anzahl der Rohre zu erhöhen. Zeckmäßigerweise sorgt man dafür, dass mindestens 10 Gew.% der gesamten Katalysatormenge als Beschichtung auf den Rippen angebracht ist. Die Katalysator-Beschichtungen weisen zumeist Schichtdicken im Bereich von 0.05 bis 5mm auf.

Die Beschichtung der Kühlrohre mit Katalysatormasse ergibt einen verbesserten Wärmeübergang vo Katalysator auf und durch die Rohrwand zum Kühlfluid. Damit lassen sich im Kühlrohrreaktor in der Hauptrealctionszone die hohen, für das bekannte Verfahren ungünstigen Reaktionstemperaturen erheblich verringern.

Ein weiterer Vorteil des Kühlrohrreaktors liegt darin, dass sich durch die Verwendung außen berippter Rohre die gasseitige Wärmeaustauschfläche in wirtschaftlicher Weise auf ein Mehrfaches der üblichen Fläche der beim bekannten Verfahren verwendeten Reaktoren vergrößern lässt. Durch eine Vergrößerung der Fläche lässt sich die Reaktionswärme leichter und mit geringerem Temperaturgefälle abführen, was zu einer zusätzlichen Erniedrigung der Reaktionstemperaturen führt. Durch Erniedrigung der Reaktionstemperaturen lässt sich bei Oxidationsreaktionen üblicherweise die Katalysator-Selektivität und damit die Produktausbeute erhöhen. Die Betriebssicherheit erhöht sich dabei in dem Maße, in dem der Abstand zwischen Katalysatortemperatur und Selbstentzündungstemperatur des Gases erhöht werden kann.

Ein weiterer erheblicher Vorteil der in den beiden vorstehenden Absätzen beschriebenen Erniedrigung der Reaktionstemperatur liegt in der Möglichkeit, die Konzentration der Einsatzstoffe im Trägergas weit über die beim bekannten Verfahren möglichen Konzentrationen ohne Gefahr der Überreaktion zu erhöhen, da die Reaktionstemperatur in ausreichend sicherem Abstand zur Selbstentzündungstemperatur des Gases gehalten werden kann. Die sich hieraus ergebende Verringerung der zu fördernden Trägergasmenge ergibt auch eine Energieeinsparung für den Betrieb des Trägergasgebläses, für die Vorwärmung des Trägergases und beim Abkühlen des Produktgemisches zur Produktabscheidung.

Eine zusätzliche Energieeinsparung ergibt sich durch die erfindungsgemäße Anordnung der Katalysatormasse auf den Rippenflächen der Kühlrohre. Dies führt zu einer erheblichen Verringerung des gasseitigen Druckverlustes über den gesamten Kühlrohrreaktor verglichen mit dem beim bekannten Vielrohrreaktor üblichen Prinzip der regellosen Füllung mit Katalysatorkörpern. Neben der beschriebenen Energieeinsparung ergibt sich auch eine Kostenminderung beim zur Förderung des Trägergases benötigten Gebläse und dessen

Antrieb; das Gebläse kann aufgrund des geringeren Förderdruckes einfacher und billiger konstruiert und gebaut werden, der Antrieb aufgrund des geringeren Energiebedarfs kleiner ausgeführt werden.

Im Kühlrohrreaktor lässt sich der Austausch von altem, verbrauchtem Katalysator gegen neuen Katalysator in kürzerer Zeit durchführen als im Vielrohrreaktor, bei dem jedes Rohr einzeln mit großer Genauigkeit gefüllt werden muss. Bei dem der Erfindung zugrunde liegenden Prinzip lassen sich die einzelnen Rohrbündel aus dem Reaktor herausziehbar ausführen, wodurch sich in zeit- und kostensparender Weise Rohrbündel mit verbrauchtem Katalysator durch neu beschichtete Rohrbündel austauschen lassen. Durch die Ziehbarkeit der Bündel erleichtert sich auch die Entfernung der verbrauchten Katalysatormasse und die darauffolgende Neubeschichtung, die ohne Stillstand des Reaktors beispielsweise im Werk des Katalysatorherstellers durchgeführt werden kann. Die Rohrbündel lassen sich leicht in Standardgröße fertigen, wodurch ermöglicht wird, dass die neu beschichteten Bündel in einem Reaktor eines anderen Anlagenbetreibers wiederverwendet werden können.

Angesichts der im Reaktor-Eintrittsteil anfallenden großen Reaktionswärme ist es für die Wirtschaftlichkeit des erfindungsgemäßen Verfahrens von erheblicher Bedeutung, die Anzahl der Kühlrohre gering zu halten. Dies gelingt z.B. durch Verwendung eines Werkstoffs mit höherer Wärmeleitfähigkeit als Stahl (z.B. Kupfer) für die Rippen in der Hauptreaktionszone. Hierdurch lassen sich relativ große Rippen von geringer Dicke einsetzen, wodurch man geringe Temperaturunterschiede zwischen Rippenrand und Kernrohr erreichen kann.

In der Nachreaktionszone des Kühlrohrreaktors, wo erheblich weniger Reaktionswärme anfällt, können dagegen Stahlrippen gleicher Abmessung wie in der Hauptreaktionszone verwendet werden. Dies hat den zusätzlichen Vorteil gleicher geometrischer Verhältnisse in allen Teilen des Reaktors.

Für die Wärmeübertragungseigenschaften eines berippten Rohres ist außer den Abmessungen und dem Werkstoff auch die Form der einzelnen Rippe von Bedeutung. Eine runde Rippe hat beispielsweise eine günstigere Temperaturverteilung als eine Rippe in quadratischer oder rechteckiger Form. Diese Form eignet sich daher insbesondere für Zonen des Reaktors mit hoher Wärmebelastung. Dagegen lassen sich bei quadratischer oder rechteckiger Form größere Wärmeaustauschflächen pro Volumeneinheit unterbringen. Dies ist wiederum vorteilhaft in Zonen mit niedriger Wärmebelastung. Bei Verwendung rechteckiger Rippen kann es vorteilhaft sein, Rippenlänge und Breite etwa im Verhältnis 2 : 1 zu wählen, so dass jede Rippe mit beiden Schenkeln des U-Rohres verbunden werden kann. Bei extrem hoher Wärmebelastung kann auch eine Rippe mit variabler Dicke von Vorteil sein, d.h. die Rippendicke ist dann nahe dem Kernrohr am größten und nimmt zum Rand hin stetig ab.

Insbesondere im Austrittsbereich des Kühlrohrreaktors ist es nicht an allen Stellen erforderlich, dass der Katalysator durch ein Kühlfluid intensiv gekühlt wird. Deshalb kann es zweckmäßig und wirtschaftlich sein, wenn sich 5 bis 40 Gew.-% der gesamten Katalysatormenge auf ungekühlten Flächen aus Metall oder anderen geeigneten Stoffen befinden. Hierbei kann es von Vorteil sein, das Reaktionsgas abwechselnd über ungekühlte und gekühlte katalytisch beschichtete Flächen zu leiten.

Einer der hauptsächlichen Vorteile bei der Verwendung eines verdampfbaren Kühlfuids im Kühlrohrreaktor gegenüber der beim bekannten Vielrohrreaktor üblichen Salzschmelze ist die gleichmäßige, nur vom Druck des Fluids abhängige Temperatur bei gleichzeitig besserem Wärmeübergang. Da die Wärmeaufnahme vorteilhafterweise durch Verdampfung und nicht durch Temperaturerhöhung wie bei der Salzschmelze geschieht, spielt die Umwälzmenge des Kühlfluids keine maßgebliche Rolle, solange jedem Kühlrohr zumindest so viel Flüssigkeit zugeleitet wird, wie zur erforderlichen Wärmeabfuhr verdampft werden muss.

Wenn das umgewälzte Kühlfluid auf die einzelnen Kühlrohre verteilt wird, kann sich das nachstehend beschriebene Verhalten des Fluids als problematisch erweisen:

Durch die unterschiedliche Menge an zu verdampfendem Kühlfluid in den Kühlrohren unterschiedlicher Reaktionszonen bilden sich unterschiedliche Druckverluste im Inneren der Kühlrohre aus, wobei in Zonen hoher Wärmeübertragung mit hoher Dampfentwicklung ein hoher Druckverlust und in Zonen niedriger Wärmeübertragung mit niedriger Dampfentwicklung ein geringer Druckverlust entsteht. Wenn alle Kühlrohre kühlmittelseitig parallel angeordnet sind, ergibt sich dadurch eine geringere Durchströmung der Rohre mit hoher Dampfentwicklung als von Rohren mit geringer Dampfentwicklung. Für das Verfahren ist aber gerade eine höhere Durchströmung von Rohren mit hoher Dampfentwicklung als bei Rohren mit geringer Dampfentwicklung vorteilhaft. Dies wird zweckmäßigerweise durch Einsatz von Drosselkörpern unterschiedlichen Strömungswiderstandes auf der Eingangs-oder Ausgangsseite eines jeden einzelnen Rohres erreicht. Dabei wird der Strömungswiderstand der Drosselkörper der erwarteten Dampfentwicklung des jeweiligen Rohres in sinnvoller Weise angepasst.

Ein weiterer Vorteil bei der Verwendung eines verdampfbaren Kühlfluids besteht darin, dass die in den Kühlrohren gebildeten Dämpfe des Kühlfluids nach Abscheidung der Flüssigphase in der Dampftrommel als Heizmedium in andern Teilen der PSA-Anlage verwendet werden können. Besonders vorteilhaft ist hierbei das hohe Temperaturniveau des zur Verfügung stehenden Dampfes. Hierdurch können aufwendige und teure Heizungseinrichtungen, z.B. eine Elektroheizung oder eine befeuertes, separates Wärmeträgersystem., welches für Temperaturen von mindestens 300 °C ausgelegt sein muss, in einfacher Weise ersetzt werden. Hierdurch ergeben sich erhebliche Einsparungen bei den Investitions- und Betriebsmittelkosten.

Durch den Anschluss des Reaktors an mehrere getrennte Kühlsysteme lassen sich die verschiedenen Reaktionen bei unterschiedlichen Temperaturen betrieben. Das Kühlfluid kann hierbei von gleicher oder unterschiedlicher Stoffzusammensetzung sein.

Der Kühlrohrreaktor kann vom Einsatzgemisch im wesentlichen horizontal oder im wesentlichen vertikal (von oben nach unten oder umgekehrt) durchströmt werden. Bei einer Ausgestaltung des Reaktors mit vertikaler Durchströmung wird Platz gespart und die Kühlrohre können als U-Rohre ohne Schweißnaht zum Gasraum ausgeführt und so im Reaktor eingeschweißt werden, dass bei der Leckage einer Schweißnaht kein Kühlfluid in den Gasraum gelangen kann.

Der Kühlreaktor kann allein oder in Serie geschaltet mit einem oder mehreren andern Reaktoren eingesetzt werden, die dem Kühlreaktors vorgeschaltet und/oder nachgeschaltet sein können. Nur beispielsweise seien hier der Vielrohrreaktor, der Wirbelschichtreaktor und Flüssigphasenreaktor genannt.

Ausgestaltungsmöglichkeiten des Verfahrens unter Verwendung eines Kühlreaktors werden mit Hilfe der Zeichnung erläutert. Es wird hierbei auf die PSA-Herstellung Bezug genommen, doch kann in analoger Weise auch ein anderer Stoff erzeugt werden.

Es zeigt:
- Fig. 1: ein Fließschema des Verfahrens,
- Fig. 2: einen Kühlrohr-Abschnitt im Längsschnitt in vergrößerter Darstellung,
- Fig. 3: ein Fließschema des Verfahrens mit zwei getrennten Kühlkreisläufen und
- Fig. 4: ein Fließschema des Verfahrens mit zwei Reaktoren.

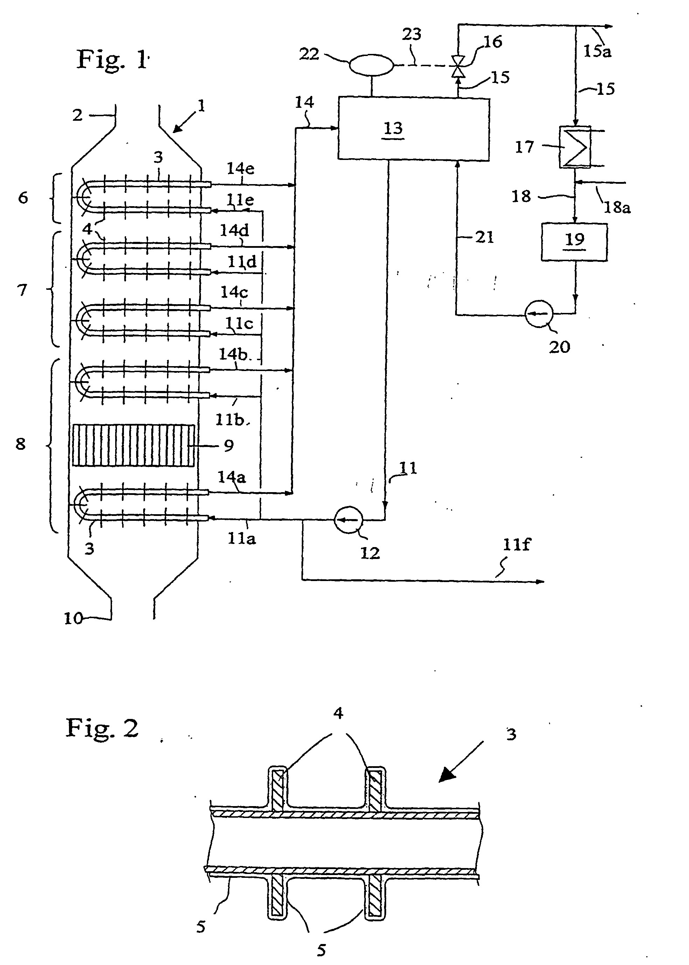

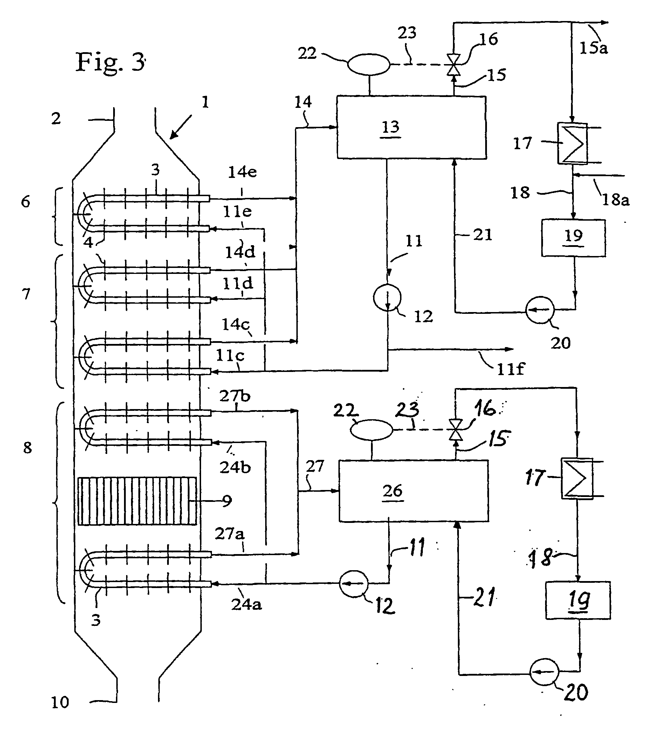

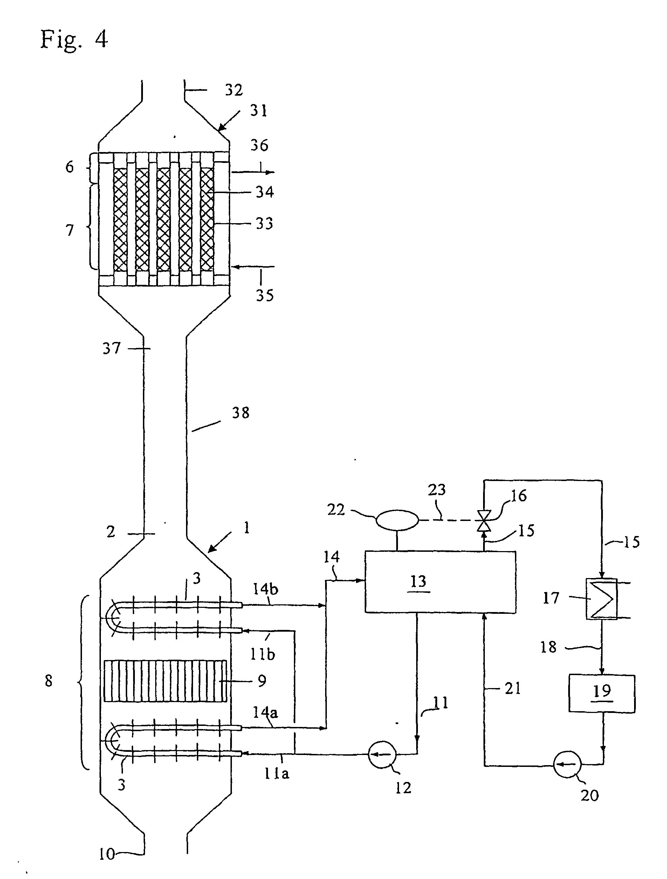

In den Kühlrohrreaktor (1) strömt gemäß Fig. 1 durch den oberen Einlass (2) das dampfförmige Einsatzgemisch, welches Orthoxylol oder Naphthalin und molekularen Sauerstoff enthält. Im Reaktor befindet sich der Katalysator (5) zu mindestens 40 Gew. -% als Beschichtung auf der Außenseite von zahlreichen Kühlrohren (3), vergleiche auch Fig. 3 und 4, auf denen ringförmige Rippen (4) angeschweißt sind. In Fig. 2 ist ein solches Rohrstück dargestellt, welches auf der Außenseite mit der punktiert angedeuteten Katalysator-Beschichtung (5) versehen ist.

Der Reaktor (1) der Fig. 1 weist eine Vorwärmzone (6), eine Hauptreaktionszone (7) und eine Nachreaktionszone (8) auf, wo jeweils etwas andere Temperaturen herrschen. Durch diese Zonen strömt das Einsatzgemisch abwärts und vorbei an den mit Katalysator beschichteten Rohren. Das dampfförmige Produktgemisch, welches PSA- Dampf enthält, verlässt den Reaktor (1) durch den Auslass (10) und wird dann in an sich bekannter, nicht dargestellter Weise (z.B. US-Patent 4 592 412) gekühlt.

Alle Zonen (6, 7, 8) weisen Bündel von Rippenrohren (3) auf, die von Kühlfluid durchströmt werden. Neben den gekühlten Rohren gibt es auch noch ungekühlte metallische oder nichtmetallische Flächen (9) mit Katalysator-Beschichtung, die vor allem zur Nachreaktionszone (8) gehören. Die Bündel der Vorwärmzone (6) im Eingangsbereich des Reaktors (1) können dagegen wahlweise katalytisch beschichtet oder unbeschichtet sein.

Die einzelnen Rohrbündel können entweder herausziehbar im Reaktor eingebaut werden oder mit diesem verschweißt sein. Die verschiedenen Teile eines Rohrbündels werden zum Beispiel unlösbar miteinander verbunden und die Katalysatorbeschichtung wird am fertigen Bündel vorgenommen, was in der Zeichnung nicht im einzelnen dargestellt ist. Der größte Teil der Länge der Kühlrohre verläuft horizontal im Reaktor entsprechend einer liegenden U-Form der Rohre, wodurch Schweißstellen im Gasraum vermieden werden. Auch wird dadurch das Problem der Thermospannungen durch Wärmedehnung weitgehend vermieden.

Das Kühlfluid kommt als Flüssigkeit aus der Leitung (11) aus einer Dampftrommel (13) und wird durch die Pumpe (12) über die Leitungen (11a bis 11 e) auf die verschiedenen Rohre verteilt. Zweckmäßigerweise tritt das Kühlfluid als Flüssigkeit von unten in die Kühlrohre (3) ein und verdampft teilweise in den Kühlrohren, wodurch die Verdampfungswärme zum Kühlen genutzt wird. Als geeignetes Kühlfluid kann zum Beispiel Diphyl verwendet werden, ein synthetisches Wärmeträgeröl. Teilweise verdampftes Kühlfluid verlässt die Rohre (3) durch die Leitungen (14a bis 14e) und strömt durch die Leitung (14) zur Dampftrommel (13).

Kühlfluid- Dampf zieht in der Leitung (15) über das Drosselventil (16) ab und wird in einem Wärmeaustauscher (17) gekühlt. Das gebildete Kondensat gelangt in der Leitung (18) zu einem Zwischenbehälter (19) und wird dann über die Pumpe (20) und die Leitung (21) zurück in die Dampftrommel (13) geführt. Eine Druckregelung (22) überwacht den Druck in der Dampftrommel (13) und hält ihn auf einem vorgegebenen Wert etwa konstant, wobei bei Bedarf über die Signalleitung (23) das Drosselventil (16) angesteuert wird. Durch den etwa konstanten Druck wird die Temperatur des flüssigen Kühlfluids in der Leitung (11) und des teilweise verdampften Kühlfluids in den Kühlrohren (3) auf der gewünschten Temperatur gehalten, die üblicherweise im Bereich von etwa 300 bis 400°C liegt, wenn Diphyl als Kühlfluid verwendet wird.

Wenn man mit zwei Reaktoren arbeitet, z.B. wenn eine Anlage erweitert wird, befindet sich der andere Reaktor entweder vor dem Einlass (2) oder nach dem Auslass (10) des Kühlrohrreaktors (1).

Fig. 3 zeigt den gleichen Reaktor wie Fig. 1, der nun jedoch an zwei getrennte Kühlkreisläufe angeschlossen ist. Hierdurch kann die Nachreaktionszone (8) des Reaktors mit Temperaturen, die unabhängig von den Temperaturen der Hauptreaktionszone (7) sind, betrieben werden. Hierbei werden die Kühlrohre (3) der Hauptreaktionszone (7) wie in Fig. 1 dargestellt, mit Kühlfluid über die Leitungen (11c bis 11e) aus der Dampftrommel (13) versorgt. Die Kühlrohre (3) der Nachreaktionszone (8) erhalten dagegen Kühlfluid aus der zweiten Dampftrommel (26), wobei die Temperaturen der Kühlflluide in beiden Dampftrommeln (13 und 26) unterschiedlich sein können. Teilweise verdampftes sowie unverdampftes Kühlfluid gelangt über die Leitungen (27a, 27b) in die Sammelleitung (27) und von dort in die Dampftrommel (26). Die Druckhaltung der Dampftrommeln (13 und 26), die Kondensation und Rückführung des kondensierten Kühlfluids geschieht in gleicher Weise wie bereits bei Fig. 1 beschrieben.

In einer weiteren Ausgestaltung des Verfahrens wird ein Teil der erzeugten Reaktionswärme für die Beheizung von Einrichtungen der PSA-Anlage genutzt, welche sich außerhalb des Reaktorbereichs befinden, beispielsweise in der thermischen Vorbehandlung und der Destillation des rohen PSA. Hierzu kann Kühlfluid wahlweise in Dampfform über die Leitung (15a) oder flüssig über die Leitung (11f) abgezogen und zu den zu beheizenden Einrichtungen geleitet werden. Der kondensierte Dampf bzw. die abgekühlte Flüssigkeit wird anschließend über die Leitung (18a) in den Reaktor- Kühlkreislauf zurückgeführt.

Fig. 4 zeigt einen ähnlichen Kühlrohrreaktor (1) wie Fig. 1, jedoch ohne die Vorwärmzone (6) und Hauptreaktorzone (7). Diesem Kühlrohrreaktor ist ein anderer Reaktor, im vorliegenden Fall ein Vielrohrreaktor herkömmlicher Bauart, vorgeschaltet, in dem die Vorwärmung des Einsatzgases und die Hauptreaktion ablaufen. Dieser andere Reaktor wird in der weiteren Beschreibung der Fig. 4 sowie im Beispiel 2 als Röhrenreaktor bezeichnet.

In diesen Röhrenreaktor (31) strömt durch den Einlass (32) das dampfförmige Einsatzgemisch, welches Orthoxylol oder Naphthalin und molekularen Sauerstoff enthält. Im Röhrenreaktor (31) befindet sich der Katalysator (34) in körniger Form im Inneren zahlreicher indirekt gekühlter Rohre (33).

Zur Wärmeabfuhr und Temperaturregelung dient ein Kühlfluid, beispielsweise eine Salzschmelze, die in den unteren Teil des Röhrenreaktors (31) über die Leitung (35) eintritt, diesen auf der Außenseite der Rohre durchströmt und über die Leitung (36) am oberen Ende wieder verlässt. Auf eine eingehende Darstellung und Beschreibung der Einrichtung zur Förderung und Rückkühlung des Kühlfluids wird an dieser Stelle verzichtet, da es bekannt ist.

Der Röhrenreaktor (31) weist eine Vorwärmzone (6) und eine Hauptreaktionszone (7) auf. Durch diese Zonen strömt das Einsatzgemisch abwärts innerhalb der mit Katalysator (34) gefüllten Rohre (33). Das dampfförmige Produktgemisch, welches PSA-Dampf enthält, dazu auch erhebliche Mengen an unreagiertem Einsatzprodukt sowie mögliche Zwischenprodukte, verlässt den Röhrenreaktor (31) durch den Auslass (37) und gelangt durch die Leitung (38) zum Kühlrohrreaktor (1) zur weiteren Umsetzung.

In den Reaktor (1) strömt durch den oberen Einlass (2) das dampfförmige Gemisch aus dem Röhrenreaktor (31). Im Reaktor (1) befindet sich der Katalysator zumindest teilweise auf der Außenseite von zahlreichen Kühlrohren (3), wie unter Fig. 1 beschrieben. Im Unterschied zu Fig. 1 weist der Reaktor (1) der Fig. 4 jedoch nur die Nachreaktionszone (8) auf, da sich die Vorwärmzone (6) und Hauptreaktionszone (7) bereits im Röhrenreaktor (31) befinden. Das dampfförmige Produktgemisch, welches PSA-Dampf enthält, aber praktisch frei ist von unreagiertem Einsatzprodukt oder möglichen Zwischenprodukten, verlässt den Reaktor (1) durch den Auslass (10) und wird dann in an sich bekannter, nicht dargestellter Weise (z.B. US-Patent 4 592 412) gekühlt.

Die Wärmeabfuhr und Temperaturregelung des Reaktors (1) der Fig. 4 geschieht in gleicher Weise wie bei Fig. 1 beschrieben mit Hilfe eines teilweise verdampften Kühlfluids.

### Beispiel 1:

Man arbeitet mit einer Verfahrensführung mit nur einem Reaktor entsprechend Fig. 1. Der Kühlrohrreaktor (1) ist ausgelegt für einen stündlichen Volumenstrom auf 180 °C vorgewärmte Luft in einer Menge von 60.000 m³ im Normzustand, die mit 6000 kg Orthoxylol als Einsatzstoff in dampfförmigem Zustand gemischt ist. Die Temperatur des in den Reaktor durch den Einlass (2) eintretenden Gasgemisches beträgt 150 °C. Das eingesetzte Orthoxylol wird in der Hauptreaktionszone (7) zu 90 % und der Rest wird in der Nachreaktionszone (8) in das Endprodukt PSA und die Nebenprodukte umgesetzt.

Der Reaktor ist senkrecht angeordnet, wobei das Gas von oben her eintritt. Der Reaktor hat eine rechteckige Form von 3200 mm Breite und 3100 mm Tiefe, die Höhe beträgt etwa 6 Meter, wobei hierbei die Bauhöhen der gasseitigen Hauben am Eintritt und Austritt mit eingeschlossen sind. Im Inneren des Reaktors sind jeweils 2 Rippenrohr-Wärmeaustauscherbündel in gleicher Höhe nebeneinander angeordnet.

Das eintretende Gasgemisch durchströmt zunächst die Vorwärmzone (6), die aus einem Paar parallel angeordneter Rippenrohr-Wärmeaustauscherbündel in U-Form aus Stahl ausgeführt ist, und wird hierbei auf 250 °C erwärmt. Die Rohre (3) der Bündel sind von flüssigem Diphyl durchströmt, das mit einer Temperatur von 370 °C in die Rohre eintritt (11e) und sich beim Durchströmen der Rohre auf etwa 320 °C am Austritt (14e) abkühlt.

Das vorgewärmte Einsatzgas durchströmt anschließend die Hauptreaktionszone (7), die aus 15 Paaren parallel angeordneter Rippenrohr-Wärmeaustauscherbündel in U-Form besteht, welche auf ihrer äußeren Oberfläche mit einer katalytischen Masse beschichtet sind. Diese besteht hauptsächlich aus Vanadiumpentoxid und Titandioxid. Beim Kontakt des Gasgemisches mit dem Katalysator reagiert das darin enthaltene Orthoxylol mit dem ebenfalls enthaltenen Sauerstoff zu PSA und anderen Nebenprodukten, wobei Reaktionswärme freigesetzt wird. Die Rohre (3) sind im Inneren von Diphyl durchströmt, welches durch Verdampfen die Reaktionswärme aufnimmt, wodurch die Katalysatortemperatur gleich gehalten werden kann. Jedes Bündel ist mit 21 parallel angeordneten U-Rohren (3) bestückt. Die U-förmigen Rippenrohre haben einen Außendurchmesser von 30 mm und eine Wandstärke von 2 mm, die gerade Rohrlänge ist 3,0 m. Die Rippen haben 60 mm Außendurchmesser und 0,5 mm Dicke. Der Werkstoff für Rippen und Kernrohre ist Kupfer. Der Abstand zweier benachbarter Rippen beträgt 1,5 mm, so dass jedes U-Rohr 3000 Rippen aufweist. Die mit einer katalytischen Beschichtung versehene Rippenoberfläche eines jeden Bündels beträgt 273,1 m².

Die bei einer erwarteten Umsetzung des eingesetzten Orthoxylols von 90 % abzuführende Wärmeleistung aller Rippenrohre der Hauptreaktionszone (7) beträgt etwa 22600 kW. Bedingt durch die erforderlichen Temperaturdifferenzen zum Wärmetransport innerhalb der Rohrwand und in der Rippe sowie zwischen Rohrwand und dem verdampfenden Diphyl steigt die Temperatur von der Rohrinnenseite zum Rippenrand hin an. Sie beträgt bei diesem Beispiel 370 °C für die verdampfende Flüssigkeit, 374 °C für die Rippe am Rippenfuß und 384 °C am Rippenrand. Durch eine günstige, jeweils um 180 °C versetzte Anordnung eines jeden Bündelpaares gegenüber dem im Gasstrom vorhergehenden und nachfolgenden beträgt die Bauhöhe der gesamten Hauptreaktionszone 1950 mm.

Das Reaktionsgasgemisch durchströmt anschließend die Nachreaktionszone (8), in welcher das noch verbliebene Orthoxylol umgewandelt wird. Die hierbei erzeugte Reaktionswärme beträgt etwa 2500 kW. Die Nachreaktionszone besteht aus 20 Rippenrohr-Wärmeaustauscherbündeln und 10 Elementen ohne Kühlung aus katalytisch beschichteten monolithischen Keramikkörpern in Wabenform (9). Die Rippenrohr-Wärmeaustauschbündel sind hierbei in Ausführung, Abmessung und Anordnung weitgehend identisch mit den für die Hauptreaktionszone (7) eingesetzten Bündeln mit den Unterschied, dass für die Nachreaktionszone die Dicke der Rippen 1 mm beträgt und die Bündel vollständig aus Stahl bestehen. Die Bündel sind wie bei der Hauptreaktionszone paarweise versetzt angeordnet. Den untersten 5 Bündelpaaren ist jeweils eine Lage ungekühlter Elemente aus Monolithkörpem nachgeschaltet.

Die Diphyl-Umwälzpumpe (12) saugt das für die einzelnen Reaktorzonen benötigte flüssige Diphyl aus einer Dampftrommel (13) an, welche in 11 m Höhe oberhalb der Pumpe aufgestellt ist. Sie fördert zu den einzelnen Reaktorzonen bedarfsgemäß unterschiedliche Mengen an flüssigem Diphyl, nämlich zur Vorwärmzone: 100 m³/h, zur Hauptreaktionszone 2000 m³/h und zur Nachreaktionszone 200 m³/h.

Das teils flüssig, teils dampfförmig aus den Bündeln der einzelnen Reaktorzonen austretende Diphyl wird über eine gemeinsame Sammelleitung (14) in die Dampftrommel (13) zurückgeführt. In der Dampftrommel werden Dampf- und Flüssigphase voneinander getrennt und die Flüssigkeit steht für eine erneute Umwälzung zur Verfügung. Der Druck in der Dampftrommel beträgt 7,5 bar, welcher der Sattdampftemperatur von 370 °C entspricht. Die über die Leitung (15) abgelassene Diphyldampfmenge beträgt 400 000 kg/h.

Das aus der Dampftrommel (13) durch die Leitung (15) abgelassene dampfförmige Diphyl wird überwiegend in mehreren Kondensatoren (17) wieder verflüssigt, im Behälter (19) aufgefangen und über die Pumpe (20) zur Dampftrommel (13) zurückgefördert.

In einem der Kondensatoren wird die Kondensationswärme zur Erzeugung von gesättigtem Wasserdampf von 51 bar verwendet. In einem hierzu parallel geschalteten weiteren Kondensator wird der so erzeugte Sattdampf auf eine Temperatur von 330 °C überhitzt und steht somit für den Antrieb einer Dampfturbine zur Verfügung. Mit der durch die Kondensation von 400.000 kg Diphyldampf stündlich übertragenen Wärme werden auf diese Weise rund 37 Tonnen überhitzten Wasserdampfes erzeugt.

Zur Beheizung der Destillation des Roh-PSA mit einem Wärmebedarf von 2000 kW wird ein weiterer Teil dampfförmigen Kühlfluids von 32.000 kg stündlicher Menge abgezogen. Das in den Wärmeverbrauchern kondensierte Kühlfluid gelangt über die Leitung (18a) zurück in das Kühlmittelsystem des Reaktors.

### Beispiel 2:

Man arbeitet mit einer Verfahrensführung entsprechend Fig. 4. Die beiden Reaktoren (31) und (1) sind ausgelegt für einen stündlichen Volumenstrom an Trägergas (vorgewärmte Umgebungsluft) von 60 000 Nm³, das mit einer Menge von 7200 kg/h Orthoxylol als Einsatzstoff in dampfförmigem Zustand beladen ist. Die Temperatur des in den Röhrenreaktor (31) durch den Einlass (32) eintretenden Gasgemisches beträgt 143 °C.

Der Röhrenreaktor (31) besitzt 15 000 senkrecht angeordnete Stahlrohre (33) von 25 mm Innendurchmesser und 3,4 m Länge. Der Katalysator (34) im Innern der Rohre hat eine Füllhöhe von 3,1 m und besteht aus ringförmigen Trägerkörpern aus inertem keramischem Material von 7 mm Durchmesser und 7 mm Länge, die mit einer dünnen Schicht aus katalytischem Material, hauptsächlich bestehend aus Vanadiumpentoxid, versehen sind.

Das als Einsatzstoff dienende Gasgemisch aus vorgewärmter Luft und dampfförmigem Orthoxylol tritt in die Rohre (33) von oben mit einer Temperatur von 144 °C ein. Beim Durchströmen des oberen, katalysatorfreien Teiles der Rohre von etwa 150 mm Länge und der obersten 100 mm der Katalysatorfüllung wird das Gas in der Vorwärmzone (6) Reaktionstemperatur von 330 °C aufgewärmt.

Durch die anschließend einsetzende Reaktion erwärmt sich das Gas weiter und gibt nach Überschreiten der Kühlfluidtemperatur von 350 °C Wärme an die Rohrwand ab. In einer Höhe von 500 mm unterhalb des Eintritts in das Katalysatorbett erreicht das Gas seine höchste Temperatur von 410 °C (hot spot). Unterhalb des hot spots kühlt sich das Gas wieder ab und erreicht beim Verlassen der Rohre (33) eine Temperatur von 360 °C.

Beim Kontakt des eingesetzten Gasgemisches mit dem Katalysator (34) in der Hauptreaktionszone (7) reagiert das darin enthaltene Orthoxylol zu PSA und anderen Nebenprodukten, wobei Reaktionswärme freigesetzt wird. Die Reaktionsbedingungen sind so beschaffen, dass etwa 10 % des eingesetzten Orthoxylols unreagiert den Röhrenreaktor (31) mit dem Reaktionsgas über die Leitung (38) verlassen.

Die bei einer erwarteten Umsetzung des eingesetzten Orthoxylols von 90 % durch das Kühlfluid aus dem Reaktor (31) abzuführende Wärmeleistung beträgt etwa 18 000 kW.

Der Kühlrohrreaktor (1) ist senkrecht angeordnet, wie in Fig. 4 dargestellt, wobei das Gasgemisch von oben her eintritt. Dieser Reaktor hat eine rechteckige Form von 3,2 m Breite und 3,1 m Tiefe, die Höhe beträgt etwa 4 m, wobei die Bauhöhen der gasseitigen Hauben am Eintritt und Austritt mit eingeschlossen sind. Im Innern des Reaktors (1) sind jeweils 2 Rippenrohr-Wärmeaustauscherbündel (3) oder 2 ungekühlte Elemente (9) auf gleicher Höhe nebeneinander angeordnet, wie weiter unten näher beschrieben.

Das Reaktionsgas aus dem Röhrenreaktor (31) umströmt im Kühlrohrreaktor (1) die Bündel (3) und die ungekühlten Elemente (9). Sowohl die Bündel als auch die Elemente sind auf ihrer Oberfläche mit einer katalytischen Masse beschichtet. Beim Kontakt des Gasgemisches mit dem Katalysator reagiert das darin enthaltene Orthoxylol und die im Röhrenreaktor (31) gebildete Zwischenprodukte mit dem ebenfalls enthaltenen Sauerstoff zu PSA und anderen Nebenprodukten, wobei Reaktionswärme freigesetzt wird. Die im Reaktor (1) erzeugte Reaktionswärme beträgt etwa 3000 kW.

Der Reaktor (1) besitzt 20 Rippenrohr-Wärmeaustauscherbündel (3) und 10 Elemente (9) gebildet aus monolithischen Keramikkörpern in Wabenform ohne Kühlung. Die U-förmigen Rippenrohre (3) der Bündel sind auf ihrer äußeren Fläche mit einer katalytischen Schicht versehen, die Monolithkörper der Elemente (9) auf der Innenseite der Waben. Die katalytische Masse besteht bei beiden vorwiegend aus Vanadiumpentoxid

Die Bündel sind paarweise angeordnet. Den untersten 5 Bündelpaaren ist jeweils eine Lage ungekühlter Elemente (9) aus Monolithkörpern nachgeschaltet, die ebenfalls paarweise angeordnet sind.

Die Rohre (3) der Bündel sind im Innern von Diphyl durchströmt, welches durch Verdampfen die Reaktionswärme aufnimmt, wodurch die Katalysatortemperatur gleich gehalten werden kann.

Jedes Bündel ist mit 21 parallel angeordneten U-Rohren (3) bestückt. Die U-förmigen Rippenrohre haben einen Außendurchmesser von 30 mm und eine Wandstärke von 2 mm, die gerade Rohrlänge beträgt 3,0 m. Die Rippen haben 60 mm Außendurchmesser und 1 mm Dicke. Der Werkstoff für Rippen und Kernrohre ist Stahl. Der Abstand zweier benachbarter Rippen beträgt 1,5 mm, so dass jedes U-Rohr 2400 Rippen aufweist. Die mit einer katalytischen Beschichtung versehene Rippenoberfläche eines jeden Bündels beträgt 218, 5 m².

Bedingt durch die erforderlichen Temperaturdifferenzen zum Wärmetransport innerhalb der Rohrwand und in der Rippe sowie zwischen Rohrwand und dem verdampfenden Diphyl steigt die Temperatur von der Rohrinnenseite zum Rippenrand hin an. Sie beträgt bei diesem Beispiel 350 °C für die verdampfende Flüssigkeit, 350,5 °C für die Rippe am Rippenfuß und 360 °C am Rippenrand.

Die ungekühlten Elemente bestehen aus katalytisch beschichteten Monolithen von 150 x 150 x 150 mm Kantenlänge in Wabenform. Da in den Waben die freigesetzte Reaktionswärme nicht abgeführt wird, erwärmt sich das Gas beim Durchströmen eines Monolithen um 1 bis 5 °C. Diese höhere Temperatur baut sich beim Durchströmen des nachfolgenden, gekühlten Bündels durch Wärmeabgabe an den berippten Rohren wieder ab.

Durch eine günstige, jeweils um 180 °C versetzte Anordnung eines jeden Bündelpaares gegenüber dem im Gasstrom vorhergehenden und nachfolgenden beträgt die Bauhöhe der gesamten Reaktionszone (8) 2,1 m.

Die Diphyl-Umwälzpumpe (12) saugt das für die Kühlung der Reaktorrohre benötigte flüssige Diphyl aus einer Dampftrommel (13) an, welche 10m Höhe oberhalb der Pumpe aufgestellt ist. Sie fördert 250 m³/h.

Das teils flüssig, teils dampfförmig aus den Bündeln über die Leitungen (14a) und (14b) austretende Diphyl wird über eine gemeinsame Sammelleitung (14) in die Dampftrommel (13) zurückgeführt. In der Dampftrommel werden Dampf- und Flüssigphase voneinander getrennt und die Flüssigkeit steht für eine erneute Umwälzung zur Verfügung. Der Druck in der Dampftrommel wird durch ein Drosselventil (16) in der Dampfaustrittsleitung (15), welches von einem Druckregler (22) über eine Impulsleitung (23) betätigt wird, konstant auf einem Druck von 5,6 bar gehalten, welcher der Sattdampftemperatur von 350 °C entspricht. Die über die Leitung (15) abgelassene Diphyldampfmenge beträgt etwa 45 000 kg/h. Der Förderdruck der Pumpe zur Überwindung der Strömungswiderstände in den Bündeln mit den eingebauten Drosselkörpern sowie den Diphyl zu- und abführenden Rohrleitungen beträgt 1 bar.

Das aus der Dampftrommel (13) durch die Leitung (15) abgelassene dampfförmige Diphyl wird überwiegend in einem Kondensator (17) wieder verflüssigt, im Behälter (19) aufgefangen und über die Pumpe (20) zur Dampftrommel (13) zurückgefördert. Hierbei wird die Kondensationswärme zur Erzeugung von gesättigtem Wasserdampf von 51 bar verwendet. Mit der durch die Kondensation von 45 000 kg/h Diphyldampf übertragene Wärme werden auf diese Weise 4 t/h Wasserdampf erzeugt.

## Patentansprüche

1. Verfahren zum katalytischen Erzeugen von organischen Stoffen durch partielle Oxidation eines organischen Einsatzstoffes in Gegenwart von molekularem Sauserstoff bei. Temperaturen in Bereich von 200 bis 500 °C in einem oder mehreren, in Serie geschalteten, einen Katalysator enthaltenden Reaktoren zur Erzeugung eines gasförmigen Produktgemisches, **dadurch gekennzeichnet, dass** mindestens ein Reaktor als Kühlrohrreaktor mit von Kühlfluid durchströmter Kühlrohren ausgebildet ist, wobei im Kühlrohrreaktor 40 bis 100 Gew. -% der Gesamtmenge des Katalysators auf der Außenseite der Kühlrohre als Beschichtung angeordnet ist und das den Einsatzstoff und den molekularen Sauerstoff enthaltende Einsatzgemisch mit den Katalysatorschichten in Kontakt kommt und dass mehrere Kühlsysteme vorhanden sind, welche Kühlfluid zu den Kühlrohren des Kühlrohrreaktors fördern und daraus abziehen.

2. Verfahren nach Anspruch 1, **dadurch gekennzeichnet, dass** im Kühlrohrreaktor mindestens die Hälfte der Kühlrohre als Rippenrohre mit an der Außenseite vorspringenden Rippen ausgebildet sind, wobei die Rippen mindestens teilweise mit Katalysator beschichtet sind.

3. Verfahren nach Anspruch 2, **dadurch gekennzeichnet, dass** mindestens 10 Gew. -% der gesamten Katalysatormenge des Kühlrohrreaktors als Beschichtung auf den Rippen aufgebracht ist.

4. Verfahren nach Anspruch 1 oder einem der folgenden, **dadurch gekennzeichnet, dass** sich 5 bis 40 Gew. -% der gesamten Katalysatormenge des Kühlrohrreaktors auf ungekühlten Metallflächen befinden.

5. Verfahren nach Anspruch 1 oder einem der folgenden, **dadurch gekennzeichnet, dass** das Einsatzgemisch im wesentlichen vertikal durch den Kühlrohrreaktor strömt und die Kühlrohre teilweise horizontal im Reaktor angeordnet sind.

6. Verfahren nach einem der Ansprüche 1 bis 5, **dadurch gekennzeichnet, dass** das Kühlfluid als Flüssigkeit in die Kühlrohre des Kühlrohrreaktors eintritt und in den Kühlrohren teilweise verdampft.

7. Verfahren nach Anspruch 1 oder einem der folgenden, **dadurch gekennzeichnet, dass** die Katalysator- Beschichtungen im Kühlrohrreaktor Schichtdicken im Bereich von 0,05 bis 5 mm aufweisen.

8. Verfahren nach Anspruch 1 oder einem der folgenden, **dadurch gekennzeichnet, dass** im Kühlrohrreaktor einer der Stoffe Phthalsäure-Anhydrid (PSA), Maleinsäureanhydrid, Acrylsäure, Essigsäure oder Anthrachinon mindestens teilweise erzeugt wird.

9. Verfahren nach Anspruch 1 oder einem der folgenden, **dadurch gekennzeichnet, dass** der Kühlrohrreaktor mit mindestens einem anderen Reaktor zusammenwirkt, der dem Kühlreaktor vor-oder nachgeschaltet ist.

10. Verfahren nach Anspruch 10, **dadurch gekennzeichnet, dass** der andere Reaktor einen Vielrohrreaktor mit einer Katalysatorschüttung in den Rohren, ein Wirbelschichtreaktor oder ein Flüssigkeitsphasenreaktor ist.

## Claims

1. A method for catalytically producing organic substances by partial oxidation of an organic feedstock in the presence of molecular oxygen at temperatures in the range from 200 to 500°C in one or more series-connected reactors containing a catalyst for producing a gaseous product mixture, **characterized in that** at least one reactor constitutes a cooling tube reactor with cooling tubes traversed by cooling fluid, where inside the cooling tube reactor 40 to 100 wt-% of the total amount of catalyst are arranged as coating on the outside of the cooling tubes, and the feed mixture containing the feedstock and the molecular oxygen gets in contact with the catalyst layers, and that several cooling systems are provided, which deliver cooling fluid to the cooling tubes of the cooling tube reactor and withdraw it therefrom.

2. The method as claimed in claim 1, **characterized in that** inside the cooling tube reactor at least half the cooling tubes constitute ribbed tubes with ribs protruding from the outside, the ribs being at least partly coated with catalyst.

3. The method as claimed in claim 2, **characterized in that** at least 10 wt-% of the entire amount of catalyst of the cooling tube reactor are applied on the ribs as coating.

4. The method as claimed in claim 1 or any of the preceding claims, **characterized in that** 5 to 40 wt-% of the entire amount of catalyst of the cooling tube reactor are provided on uncooled metal surfaces.

5. The method as claimed in claim 1 or any of the preceding claims, **characterized in that** the feed mixture flows through the cooling tube reactor substantially vertically and the cooling tubes are in part horizontally arranged in the reactor.

6. The method as claimed in any of claims 1 to 5, **characterized in that** the cooling fluid enters the cooling tubes of the cooling tube reactor as liquid and partly evaporates in the cooling tubes.

7. The method as claimed in claim 1 or any of the preceding claims, **characterized in that** the catalytic coatings in the cooling tube reactor have layer thicknesses in the range from 0.05 to 5 mm.

8. The method as claimed in claim 1 or any of the preceding claims, **characterized in that** in the cooling tube reactor one of the substances from the group including phthalic anhydride, maleic anhydride, acrylic acid, acetic acid or anthraquinone is at least partly produced.

9. The method as claimed in claim 1 or any of the preceding claims, **characterized in that** the cooling tube reactor cooperates with at least one other reactor provided upstream or downstream of the cooling tube reactor.

10. The method as claimed in claim 10, **characterized in that** the other reactor is a multi-tube reactor with a catalyst bed in the tubes, a fluidized-bed reactor or a liquid-phase reactor.

## Revendications

1. Procédé de production catalytique de matières organiques par oxydation partielle d'une matière de charge organique en présence d'oxygène moléculaire à une température comprise entre 200 et 500°C dans un ou plusieurs réacteurs montés en série et contenant un catalyseur, pour produire un mélange de produit gazeux, **caractérisé en ce qu'**au moins un réacteur est formé en tant qu'un réacteur à tubes de refroidissement comportant des tubes de refroidissement parcourus par du fluide de refroidissement, dans lequel, dans le réacteur à tubes de refroidissement, 40 à 100 % en poids de la quantité totale du catalyseur sont disposés sur le côté extérieur du tube de refroidissement en tant que revêtement et le mélange de charge contenant la matière de charge et l'oxygène moléculaire vient en contact avec les couches de catalyseur, et **en ce que** plusieurs systèmes de refroidissement sont prévus, lesquels acheminent du fluide de refroidissement vers les tubes de refroidissement du réacteur à tubes de refroidissement et extraient du fluide de refroidissement de ces tubes.

2. Procédé suivant la revendication 1, **caractérisé en ce que**, dans le réacteur à tubes de refroidissement, au moins la moitié des tubes de refroidissement est réalisée sous la forme de tubes nervurés comportant des nervures faisant saillie du côté extérieur, les nervures étant recouvertes au moins en partie de catalyseur.

3. Procédé suivant la revendication 2, **caractérisé en ce qu'**au moins 10 % en poids de la quantité totale de catalyseur du réacteur à tubes de refroidissement sont déposés sur les nervures en tant que revêtement.

4. Procédé suivant la revendication 1 ou l'une des suivantes, **caractérisé en ce que** de 5 à 40 % en poids de la quantité totale de catalyseur du réacteur de tubes de refroidissement se trouvent sur des surfaces métalliques non refroidies.

5. Procédé suivant la revendication 1 ou l'une des suivantes, **caractérisé en ce que** le mélange de charge s'écoule sensiblement verticalement dans le réacteur à tubes de refroidissement et les tubes de refroidissement sont disposés dans le réacteur partiellement horizontalement.

6. Procédé suivant l'une des revendications 1 à 5, **caractérisé en ce que** le fluide de refroidissement entre en tant que liquide dans les tubes de refroidissement du réacteur à tubes de refroidissement et se vaporise partiellement dans les tubes de refroidissement.

7. Procédé suivant la revendication 1 ou l'une des suivantes, **caractérisé en ce que** les revêtements de catalyseur ont une épaisseur de couche dans le réacteur de tubes de refroidissement comprise entre 0,05 mm et 5 mm.

8. Procédé suivant la revendication 1 ou l'une des suivantes, **caractérisé en ce que** plusieurs systèmes de refroidissement sont présents, lesquels transportent du fluide de refroidissement vers les tubes de refroidissement du réacteur à tubes de refroidissement et l'en extraient.

9. Procédé suivant la revendication 1 ou l'une des suivantes, **caractérisé en ce que**, dans le réacteur à tubes de refroidissement, il est produit au moins partiellement l'une des matières que sont l'anhydride d'acide phtalique (PSA), l'anhydride d'acide maléique, l'acide acrylique, l'acide acétique ou l'anthraquinone.

10. Procédé suivant la revendication 1 ou l'une des suivantes, **caractérisé en ce que** le réacteur à tubes de refroidissement coopère avec au moins un autre réacteur, qui est monté en amont ou en aval du réacteur de refroidissement.
